(19) Europäisches Patentamt
European Patent Office
Office européen des brevets

(11) Veröffentlichungsnummer: **0 276 653 B1**

(12) # EUROPÄISCHE PATENTSCHRIFT

(45) Veröffentlichungstag der Patentschrift: **21.08.91**

(51) Int. Cl.5: **C07C 69/757**, C07C 67/32

(21) Anmeldenummer: **88100089.7**

(22) Anmeldetag: **07.01.88**

(54) **Verfahren zur Herstellung von Bicyclo [3.3.0]octan-3,7-dion-2-carbonsäure-estern.**

(30) Priorität: **23.01.87 DE 3702385**

(43) Veröffentlichungstag der Anmeldung:
**03.08.88 Patentblatt 88/31**

(45) Bekanntmachung des Hinweises auf die Patenterteilung:
**21.08.91 Patentblatt 91/34**

(84) Benannte Vertragsstaaten:
**AT BE CH DE ES FR GB GR IT LI LU NL SE**

(56) Entgegenhaltungen:

**THE JOURNAL OF ORGANIC CHEMISTRY, Band 45, Nr. 23, 7. November 1980, Seiten 4776-4778, American Chemical Society, Columbus, Ohio, US; A. BARCO et al.: "A new, elegant route to a key intermediate for the synthesis of 9(O)-methanoprostacyclin"**

**THE JOURNAL OF ORGANIC CHEMISTRY, Band 42, Nr. 18, 2. September 1977, Seiten 3089-3091, American Chemical Society, Columbus, Ohio, US; S.P. BHATNAGAR et al.: "Reaction of dimethyl 3-ketoglutarate with 1,2-dicarbonyl compounds. 8. Selective base-catalyzed decarbomethoxylation of tetramethyl 3,7-dioxo-cis-bicyclo[3.3.0]octane-2,4,6,8-tetracarboxylate. Preparation of 2,6-dicarbomethoxy-cis-bicyclo[3.3.0]octane-3,7-dione"**

(73) Patentinhaber: **SCHERING AKTIENGESELLSCHAFT Berlin und Bergkamen
Müllerstrasse 170/178 Postfach 65 03 11
W-1000 Berlin 65(DE)**

(72) Erfinder: **Vorbrüggen, Helmut, Prof.
Wilkestrasse 7
W-1000 Berlin 27(DE)**
Erfinder: **Krolikiewicz, Konrad
Neuhofer Strasse 75
W-1000 Berlin 47(DE)**

**EP 0 276 653 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Bicyclo [3.3.0]octan-3,7-dion-2-carbonsäureestern und dazugehöriger Monoketale.

Bicyclo [3.3.0]octan-3,7-dion-2-carbonsäureester sind wichtige potentielle Zwischenprodukte für die Herstellung von Carbacyclin-Derivaten und Sesquiterpenen, die bisher nur schwer zugänglich sind.

Die Spaltung von D,L-Bicyclo [3.3.0]octan-3,7-dion-2,6-dicarbonsäurediestern der Formel II,

R = $CH_3$, $C_2H_5$

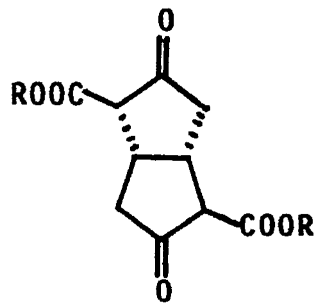

(II),

mit Natriummethylat in DMSO-Methanol [nach U. Weiss et al., J.Org.Chem.42, 3089 (1977)] ergibt nur Spuren an D,L-Bicyclo [3.3.0]octan-3,7-dion-2-carbonsäureestern.

R = $CH_3$, $C_2H_5$

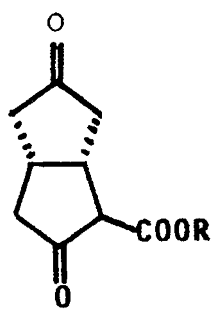

Es wurde nun überraschend gefunden, daß sich D,L-Bicyclo-[3.3.0] octan-3,7-dion-2-carbonsäureester in Ausbeuten von 50 - 70 % herstellen lassen, wenn man D,L-Bicyclo [3.3.0] octan-3,7-dion-2,6-dicarbonsäureester der Formel II in wäßrigem oder Wasser enthaltendem Medium in Gegenwart von Säuren partiell verseift und decarboxyliert.

Gegenstand der Erfindung ist also die selektive Spaltung der D,L-Diester II unter milden Säurebedingungen zu D,L-Bicyclo-[3.3.0] octan-3,7-dion-2-carbonsäureester.

Das Verfahren zur Herstellung von D,L-Bicyclo [3.3.0]octan-3,7-dion-2-carbonsäureestern der Formel I

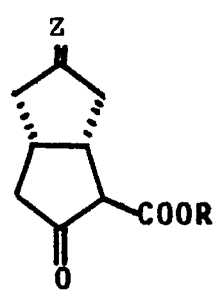

(I),

worin

R Methyl oder Ethyl und

Z Sauerstoff oder den Ketal-Rest

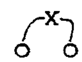

mit X in der Bedeutung ethylen, trimethylen oder 2,2-dimethyl-trimethylen darstellen, ist dadurch gekennzeichnet, daß man D,L-Bicyclo [3.3.0] octan-3,7-dion-2,6-dicarbonsäureester der Formel II

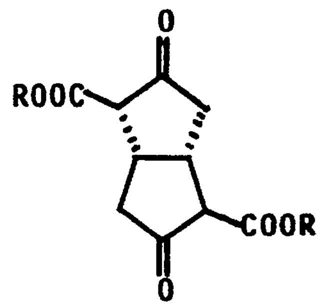

(II),

worin R die oben angegebene Bedeutung hat, in wäßrigem oder Wasser enthaltendem Medium in Gegenwart von Säuren partiell verseift und decarboxyliert und gegebenenfalls selektiv ketalisiert.

Die Ausgangsverbindungen der Formel II sind durch Alkalibehandlung der Bicyclo [3.3.0]octan-3,7-dion-2,4,6,8-tetracarbonsäureester III [vgl. J.Org. Chem. 42, 3089 (1977); Org. Synth. 64, 27 - 37 (1985)] sehr leicht zugänglich.

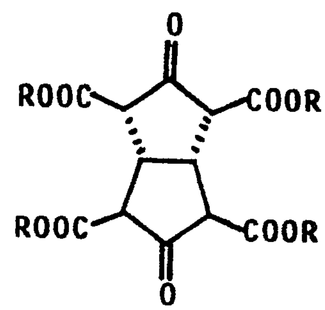

(III),

Die saure Spaltung der D,L-Diester der Formel II zu den D,L-Monoestern der Formel I wird vorzugsweise in $H_2O$ mit wasserlöslichen Säuren bzw. Säuregemischen, die möglichst unlöslich in Methylenchlorid sein sollen, in Mengen von 0,05 - 1 Mol bei einem pH-Wert von 1 - 6, vorzugsweise von 3 - 4, und Temperaturen von 20 - 150 °C, vorzugsweise bei 80 - 100° durchgeführt. Bei anschließender Abkühlung auf 2 °C kristallisiert der nicht umgesetzte D,L-Diester II aus, der so größtenteils abgetrennt und zurückgewonnen wird. Extraktion des Filtrats mit Methylenchlorid ergibt dann ein Rohprodukt, aus dem reiner D,L-Monomethylester der Formel I (R = $CH_3$, Z = 0) auskristallisiert.

Während der Tetracarbonsäureethylester III (R = $C_2H_5$), Schmpt. 107 °C, sowie der D,L-Dicarbonsäureethylester II (R = $C_2H_5$), Schmpt. 102,7 °C kristallin sind, ist der D,L-Diketonmonoethylester I (R = $C_2H_5$) ölig, so daß diese Verbindung nur chromatographisch rein erhalten werden kann und deshalb als Zwischenprodukt im Vergleich zum kristallinen D,L-Diketonmonomethylester (R = $CH_3$) eine geringere Bedeutung hat.

Als wasserlösliche Säuren für die Abspaltung der Estergruppe lassen sich Citronensäure, Milchsäure, Oxalsäure, Essigsäure, Ameisensäure, Asparaginsäure, Glutaminsäure, $NaH_2PO_4$, Amidosulfonsäure usw., vorzugsweise Citronensäure,einsetzen. Bei Einsatz von Ameisensäure und Essigsäure, die als Lösungsmittel in größeren Mengen zugesetzt werden, lassen sich diese durch Abdampfen im Vakuum sehr leicht entfernen.

Zur Verkürzung der Reaktionszeit sollte möglichst bei Temperaturen von 50-90 °C gearbeitet werden.

Da die ß-Ketoester-Systeme im D,L-Diester II schwache Säuren sind (inherente Acidität), läßt sich II in Wasser oder in azeotropen Gemischen mit organischen Lösungsmitteln auch ohne Zusatz von Carbonsäuren, aber dann mit verminderter Reaktionsgeschwindigkeit in I umwandeln. So ergibt II (R = $CH_3$) durch 1,5 h Kochen in Xylol-$H_2O$ bei 120 - 140° C ca. 41 % I (R = $CH_3$).

Die D,L-Monoester I (Z = O) lassen sich selektiv in die D,L-Monoketale I

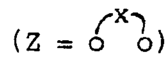

überführen.

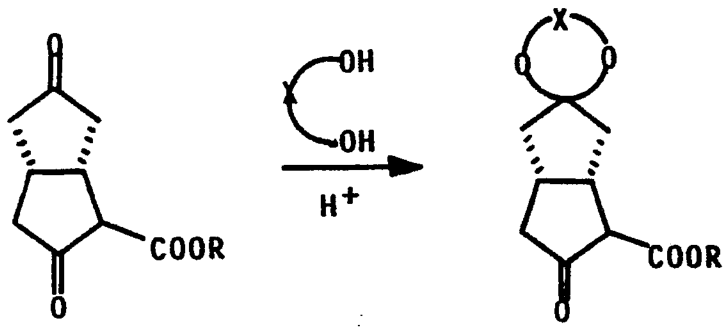


X = $-(CH_2)_2$; $-(CH_2)_3$ bzw. 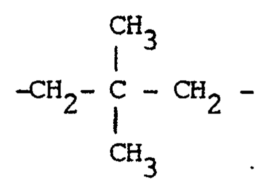

Diese Monoketale sind vorzügliche Ausgangsmaterialien für die Darstellung von Carbacyclinen und wurden bisher nach K.C. Nicolaou et al., J.Chem.Soc., Chem. Comm. 433 (1979) durch Carboäthoxylierung der Monoketale des Bicyclo[3.3.0]octan-3,7-dions hergestellt.

Beispiel 1:

Darstellung von D,L-Bicyclo [3.3.0] octan-3,7-dion-2-carbonsäuremethylester I (R = $CH_3$) aus D,L-Bicyclo [3.3.0] -octan-3,7-dion-2,6-dicarbonsäuredimethylester II (R = $CH_3$).

a) mit Citronensäure

105 g (0,5 Mol) Citronensäuremonohydrat werden in 1,3 l dest. $H_2O$ gelöst und im Ölbad auf 85 - 90° C (Innentemperatur) erhitzt. Der feingepulverte Dimethylester (127 g = 0,5 Mol) wird auf einmal zugegeben und die entstehende Suspension lebhaft gerührt, wobei sich innerhalb von 30 min. eine klare Lösung bildet. Nach weiteren 90 min. bei 85 - 90° C Innentemperatur im Kolben wird möglichst schnell mit Eiswasser auf 2° C Innentemperatur im Kolben abgekühlt, wobei nicht umgesetzter Diester II als farblose Substanz auskristallisiert. Nach ca. 30 min. bei 2° C wird filtriert und mit 500 ml Eiswasser nachgewaschen. Man gewinnt so nach Trocknen bei 40 - 50° C im Vakuumtrockenschrank 33,6 g (Schmpt. 103° C) = 26,3 % der eingesetzten Menge an reinem Diester II zurück.

Falls diese Reaktion mit größeren Mengen II und daher in größeren Volumina wässriger Citronensäure durchgeführt wird und sich diese Lösung aus technischen Gründen nur relativ langsam auf 2° C abkühlen läßt, muß die Reaktionszeit von 120 min. bei 85 - 90° C auf 60 - 90 min. verkürzt werden, da die Esterspaltung bei langsamerem Abkühlen noch weiter läuft.

Das eiskalte Filtrat wird mit 3 x 300 ml $CH_2Cl_2$ extrahiert, getrocknet ($Na_2SO_4$) und eingeengt. Der Rückstand (68 g) wird in 250 ml kochendem Methyl-t-butyläther gelöst und langsam auf 24° C abgekühlt, wobei 28 g praktisch reiner Diketon-Monomethylester I (Schmpt. 67° C) auskristallisiert. (Die DC zeigt nur noch Spuren Diester). Nach Abdampfen des Lösungsmittels wird der Rückstand in 150 ml eiskalter 2 NaOH gelöst, wobei die Lösung stark alkalisch (pH = 13) werden muß. Nach Extraktion mit 3 x 250 ml $CH_2Cl_2$ und Trocknen der Extrakte ($Na_2SO_4$) werden beim Abdampfen ca. 10,4 g (15 %) Diketon IV erhalten (bezogen auf den eingesetzten Diester II).

Die alkalische Lösung wird mit ges. wässriger Citronensäure auf pH 3 angesäuert und mit 3 x 300

$CH_2Cl_2$ extrahiert. Nach Trocknen ($Na_2SO_4$) und Abdampfen erhält man 29 g gelben öligen Rückstand. Der Rückstand wird in $CH_2Cl_2$ (50 ml) gelöst und über eine kurze Säule von eisenfreiem Silikagel (0,063 - 0,2 mm) filtriert und mit insgesamt 1000 ml $CH_2Cl_2$ nachgewaschen. Abdampfen des Filtrats ergibt 26,5 g Rückstand, aus dem nach Kristallisation aus 150 ml Methyl-t-butyläther 16,5 g Diketon-Monoester I erhalten werden. Die Mutterlauge wird nach Abdampfen in 30 ml Toluol gelöst und an 100 g eisenfreiem Silikagel (0,063 - 0,2 mm) chromatographiert.

Fraktionen 1 + 2 je 250 ml Toluol
Fraktion 3 250 ml Toluol/Essigester 95 : 5 2,4 g Diester II
Fraktionen 4 - 8 je 250 ml Toluol/Essigester 95 : 5 5,9 g Diketon-Monoester I
Fraktionen 9 - 12 je 250 ml Toluol/Essigester 95 : 5 1,4 g Diketon IV
Kristallisation der Fraktionen 4 - 8 aus 20 ml Methyl-t-butyläther ergibt 5,1 g Diketon-Monoester I .

Gesamtausbeute an Diketon-Monoester I : 49,6 g = 50 % bezogen auf eingesetzten Diester II, 70,5 % bezogen auf umgesetzten Diester II.

b) mit Ameisensäure

2,54 g (0,01 Mol) feingepulverter Dimethylester II werden in einem Gemisch von 3 ml 98 - 100 I% Ameisensäure und 7 ml $H_2O$ bei 76° C Innentemperatur 4,5 Stunden gerührt, wobei II sich löst. Nach ca. 4 Stunden Erhitzen wird auf 22° C abgekühlt und schließlich 30 min. auf 2° C abgekühlt und der auskristallisierte Diester II (0,46 g = 16,9 % Schmpt. 108° C) abgefiltert. Das Filtrat wird zweimal bei 40° C im Vakuum mit jeweils 5 ml $H_2O$ abgedampft und der Rückstand aus 6 ml Methyl-t-butyläther kristallisiert, wobei in zwei Portionen ca. 0,64 g I, Schmpt. 66° C (32 % bezogen auf eingesetztes II, 39,3 % bezogen auf umgesetztes II) erhalten werden. Die Mutterlaugen enthalten nach der DC-Analyse noch weiteren Monoester.

c) mit Essigsäure

2,54 g (0,01 Mol) feingepulverter Dimethylester II wurde 4,5 Stunden in einem Gemisch von 3 ml Essigsäure und 7 ml $H_2O$ erhitzt. Nach Abkühlen über Nacht auf 22° C und Herunterkühlen für 1 Stunde auf 2° C wurden 0,68 g (26, 77 %) II, Schmpt. 108° C abfiltriert und zweimal nach Zusatz von jeweils 5 ml $H_2O$ abgedampft. Der farblose Rückstand (1,3 g) ergab bei Kristallisation aus 4 ml Methyl-t-butyläther 0,58 g III, Schmpt. 66,4° C (26,9 % bezogen auf eingesetztes II, 40 % bezogen auf umgesetztes II).

d) in Xylol-$H_2O$

2,54 g (0,01 Mol) feingepulverter Diester II (R = $CH_3$) wurde in 30 ml Xylol und 10 ml $H_2O$ bei 160° C Ölbadtemperatur 1,5 h am Rückfluß gekocht, wobei die Temperatur im Kolben allmählich von 120° C auf 140° C anstieg.

Nach Abdestillieren des Xylols im Vakuum bei 35° C wurde der Rückstand (2,7 g) in 15 ml Toluol gelöst und an einer Säule mit 80 g eisenfreiem Silicagel chromatographiert. Nach einem Vorlauf mit 500 ml Toluol eluierten 300 ml Toluol-Essigester-Gemisch (98 : 2) 0,280 g (11 %) kristalliner Diketon-dimethylester II (R = $CH_3$). Weitere Elution mit 300 ml Toluol-Essigester-Gemisch (95 : 5) lieferte 0,80 g (40,8 %) kristallinen Diketonmonomethylester I (R = $CH_3$). Elution mit 300 ml Essigester-Toluol ergab schließlich 0,6 g (43 %) Diketon IV.

e) in $H_2O$

Die Reaktion von reinem Diester II (R = $CH_3$) in $H_2O$ bei 85 - 90° C verläuft analog zu 1d), benötigt aber ca. 5 - 6 Stunden, um zur analogen Ausbeute von Monoester I (R = $CH_3$) zu führen.

Beispiel 2:

Darstellung des Neopentylketals des Bicyclo [3.3.0] octan-3,7-dion-2-carbonsäuremethylesters I (R = $CH_3$, Z = $-O-CH_2-C(CH_3)_2-CH_2-O-$).

1,0 g (5,01 mMol) D,L-Bicyclo[3.3.0]octan-3,7-dioncarbonsäuremethylester werden mit 783 mg (7,52 mMol) Neopentylglycol (MERCK AG) und 58 mg (0,25 mMol) DL-Camphersulfonsäure (Fluka) und 1,0 g wasserfreiem $MgSO_4$ (Merck AG) in 8 ml $CH_2Cl_2$ 4 h bei 22 - 24° C gerührt, wonach die DC-Probe im System Hexan-Diäthyläther (4 - 6) neben Spuren Ausgangsmaterial als überwiegendes Hauptprodukt die

Bildung des gewünschten D,L-Monoketals sowie geringe Mengen des Diketals anzeigt. Nach Aufarbeiten mit gesättigter $NaHCO_3$-Lösung wird die vereinigte $CH_2Cl_2$-Lösung getrocknet ($MgSO_4$) und abgedampft. Der Rückstand (1,46 g) wird über eine Säule von 10 g eisenfreiem Silicagel in Hexan - Äther (8 : 2) filtriert. Nach Abdampfen kristallisiert das Filtrat (1,21 g) aus ca. 4 ml heißem Hexan.
Nach Stehen über Nacht bei 24° C werden 1,08 g (75%) reines D,L-Monoketal, Schmpt. 68 - 70° C erhalten. Nach Abdampfen des Filtrats ist im Rückstand (0,080 g) das Diketal deutlich angereichert.

Referenz-Beispiel:

Darstellung des D,L-Bicyclo [3.3.0] octan-3,7-dion-2,6-dicarbonsäuredimethylesters II.

In einer eiskalten Lösung von 80 g (2 Mol) NaOH in 1,5 l Methanol werden 348,3 g (2 Mol) Acetondicarbonsäuremethylester innerhalb von 30 Minuten unter Kühlung zugetropft, wobei ein gelber Niederschlag ausfällt. Anschließend wird die Methanollösung unter Rühren zum Sieden erhitzt, wobei sich der Niederschlag löst. Zur heißen Lösung tropft man während 1 Stunde 164,2 g (1,13 Mol) einer 40 % Lösung von Glyoxal in $H_2O$ wobei die Reaktionsmischung am Sieden bleibt. Anschließend läßt man auf Zimmertemperatur abkühlen und kühlt schließlich auf 4° C. Nach 1 h bei 4° wird abfiltriert und das Salz mit insgesamt 600 ml Methanol gewaschen, bis das Filtrat farblos wird. Das gelbliche Dinatriumsalz des Tetraesters III wird in 2 l $H_2O$ suspendiert und eine Lösung von 120 g (3 Mol) NaOH in 500 ml $H_2O$ zugegeben. Nach ca. 18 Stunden bei 22° C wird die klare Lösung portionsweise mit 195 g Citronensäure-monohydrat under $CO_2$-Entwicklung auf ca. pH = 8,5 angesäuert, wobei der Diester II anfängt zu kristallisieren. Nach Abkühlen und einstündigem Stehen bei 3° C wird der Diester II abfiltriert, mit 500 ml $H_2O$ gewaschen und bei 40° im Vakuum getrocknet. Ausbeute 154 g (60,4 % bezogen auf Acetondicarbonsäuredimethylester).

## Patentansprüche

1. Verfahren zur Herstellung von D,L-Bicyclo [3.3.0] octan-3,7-dion-2-carbonsäureestern der Formel I

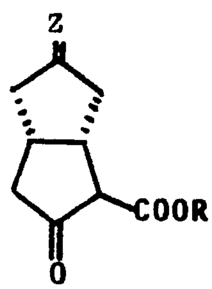

(I),

worin

R Methyl oder Ethyl und
Z Sauerstoff oder den Ketal-Rest

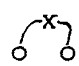

mit X in der Bedeutung ethylen, trimethylen oder 2,2-dimethyl-trimethylen darstellen,

dadurch gekennzeichnet, daß man D,L-Bicyclo [3.3.0] octan-3,7-dion-2,6-dicarbonsäureester der Formel II

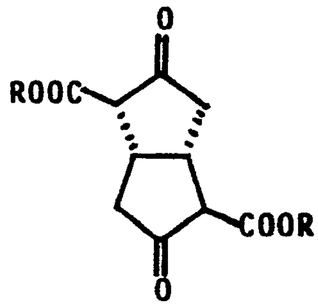

(II),

worin R die oben angegebene Bedeutung hat, in wäßrigem oder Wasser enthaltendem Medium in Gegenwart von Säuren partiell verseift und decarboxyliert und gegebenenfalls selektiv ketalisiert.

2. Verfahren nach Anspruch 1, dadurch gekennzeichnet, daß man als Säuren wasserlösliche, flüchtige Säuren oder wasserlösliche, nichtflüchtige und in Methylenchlorid unlösliche Säuren verwendet.

3. Verfahren nach Anspruch 2, dadurch gekennzeichnet, daß man als flüchtige Säuren Ameisensäure oder Essigsäure verwendet.

4. Verfahren nach Anspruch 2, dadurch gekennzeichnet, daß man als nichtflüchtige Säuren Citronensäure oder Glutaminsäure verwendet.

**Claims**

1. Process for the preparation of D,L-bicyclo[3.3.0]octane-3,7-dione-2-carboxylic acid esters of formula I

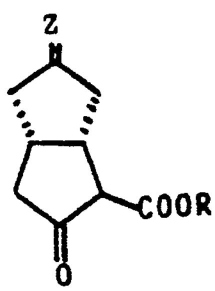

(I),

wherein

R represents methyl or ethyl and

Z represents oxygen or the ketal radical

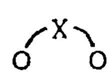

in which X represents ethylene, trimethylene or 2,2-dimethyltrimethylene,

characterised in that D,L-bicyclo[3.3.0]octane-3,7-dione-2,6-dicarboxylic acid esters of formula II

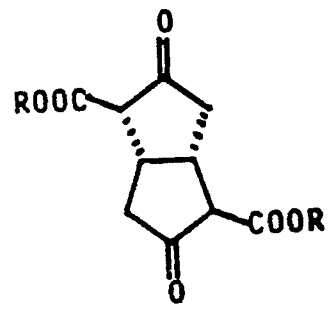

(II),

wherein R has the meaning indicated above, are partially hydrolysed and decarboxylated in aqueous or water-containing medium in the presence of acids and, optionally, selectively ketalised.

2. Process according to claim 1, characterised in that water-soluble volatile acids or acids that are water-soluble, non-volatile and insoluble in methylene chloride are used as the acids.

3. Process according to claim 2, characterised in that formic acid or acetic acid are used as the volatile acids.

4. Process according to claim 2, characterised in that citric acid or glutamic acid are used as the non-volatile acids.

**Revendications**

1. Procédé pour préparer des esters de l'acide dioxo-3,7 bicyclo[3.3.0]octane-carboxylique-2 D,L répondant à la formule I :

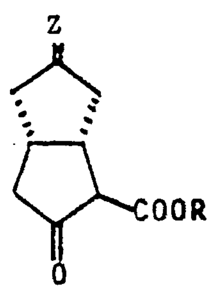

(I)

dans laquelle :

R représente un radical méthyle ou éthyle et

Z représente l'oxygène ou un radical acétal

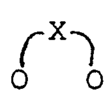

dans lequel X représente un radical éthylène, triméthylène ou diméthyl-2,2 triméthylène,

procédé caractérisé en ce qu'on saponifie partiellement et décarboxyle, en présence d'acides, dans un milieu aqueux ou un milieu contenant de l'eau, un ester de l'acide dioxo-3,7 bicyclo[3.3.0]octane-dicarboxylique-2,6 D,L répondant à la formule II :

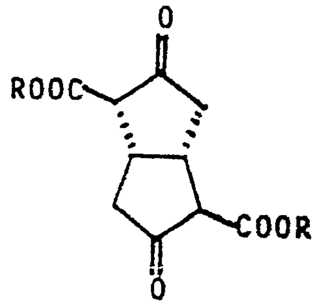

(II)

dans laquelle R a la signification qui lui a été donnée ci-dessus.

2. Procédé selon la revendication 1 caractérisé en ce qu'on utilise, comme acides, des acides volatils solubles dans l'eau ou des acides non volatils, solubles dans l'eau et insolubles dans le chlorure de méthylène.

3. Procédé selon la revendication 2 caractérisé en ce qu'on utilise, comme acide volatil, l'acide formique ou l'acide acétique.

4. Procédé selon la revendication 2 caractérisé en ce qu'on utilise, comme acide non-volatil, l'acide citrique ou l'acide glutamique.